# EUROPÄISCHE PATENTANMELDUNG

(11) **EP 1 357 166 A1**
(43) Veröffentlichungstag der Anmeldung: **29.10.2003**
(21) Anmeldenummer: 03007551.9
(22) Anmeldetag: 01.04.2003
(51) Int. Cl.: C10G 51/02, C10G 51/04, C10G 55/02, C10G 55/04, C10G 55/06, C10G 7/08, C10G 9/20, C10G 11/00, C07C 7/10

(54) **Verfahren und Vorrichtung zur Olefinherstellung**

(30) Priorität: 22.04.2002 DE 10217863
(71) Anmelder: Linde Aktiengesellschaft, 65189 Wiesbaden (DE)
(72) Erfinder: Bölt, Heinz, 82515 Wolfratshausen (DE); Fritz, Helmut, 81373 München (DE); Glanz, Stephan, Dr., 85579 Neubiberg (DE)

(57) **Zusammenfassung**

Es werden ein Verfahren und eine Vorrichtung zur Olefinherstellung beschrieben, wobei ein kohlenwasserstoffhaltiger Einsatz, z.B. Naphtha, in einer Behandlungsanlage (1), insbesondere einer Raffinerie oder Olefinanlage, in gewünschte Olefine, z.B. Ethylen und Propylen, umgesetzt wird. Dabei wird zumindest ein Teil der in der Behandlungsanlage (1) anfallenden Fraktionen, die längerkettige, insbesondere mindestens vier Kohlenstoffatome aufweisende, Olefine enthalten, einer Olefinkonversionsstufe (12) zugeführt. In der Olefinkonversionsstufe (12) wird zumindest ein Teil der längerkettigen Olefine in kürzerkettige Olefine umgesetzt. Als Olefinkonversionsstufe kann beispielsweise ein katalytischer Reaktor, insbesondere ein Festbettreaktor, eingesetzt werden. Um die Ausbeute an den gewünschten Olefinen zu erhöhen, wird vorgeschlagen, der Olefinkonversionsstufe (12) eine Paraffin-Olefin-Trennstufe (23) vorzuschalten. In dieser Paraffin-Olefin-Trennstufe (23) wird insbesondere durch Extraktivdestillation eine Auftrennung in Paraffine, z. B. Butan und Pentan, sowie Olefine, z. B. Buten und Penten, durchgeführt. Während die so gewonnenen Paraffine zur Behandlungsanlage (1), z. B. zum Spaltofen (3) der Olefinanlage, zurückgeführt werden, werden die Olefine zur Olefinkonversionsstufe (12) geführt.

## Beschreibung

Die Erfindung betrifft ein Verfahren zur Olefinherstellung aus einem kohlenwasserstoffhaltigen Einsatz, wobei der Einsatz einer Behandlungsanlage, insbesondere einer Raffinerie oder Olefinanlage, zugeführt wird, in der verschiedene kohlenwasserstoffhaltige Fraktionen erzeugt werden, wobei zumindest ein Teil der Fraktionen, die längerkettige, insbesondere mindestens vier Kohlenstoffatome aufweisende, Olefine enthalten, einer Olefinkonversionsstufe zugeführt wird, in der zumindest ein Teil der längerkettigen Olefine in kürzerkettige Olefine umgesetzt wird, welche zumindest zum Teil zur Behandlungsanlage zurückgeführt werden, sowie eine Vorrichtung zur Durchführung des Verfahrens.

Der Markt für chemische Grundstoffe lässt einen steigenden Bedarf an Propylen in den letzten Jahren erkennen. Bisher wurde Propylen hauptsächlich als Nebenprodukt in Olefinanlagen, insbesondere Ethylenanlagen, erzeugt. Da der zukünftige Bedarf an Propylen auf diesem Weg allein nicht mehr zu decken sein wird, wurden bereits Verfahren entwickelt, um die Propylenausbeute in Olefinanlagen zu erhöhen. Diese Verfahren beruhen im Wesentlichen auf dem Prinzip, die in Olefinanlagen anfallenden längerkettigen Olefine mit z.B. vier bis sechs Kohlenstoffatomen in kürzerkettige Olefine mit z.B. zwei und drei Kohlenstoffatomen, insbesondere in Propylen (drei Kohlenstoffatome) umzuwandeln. Hierzu wird üblicherweise ein Katalysator verwendet, der die entsprechende Umsetzung der Olefine bewirkt. Derartige Verfahren sind unter den Namen "Propylur-Verfahren" (siehe Hydro Carbon Engineering, May 1999, Seite 66 bis 68) und "Meta-4-Verfahren" (siehe Petrochemicals PTQ Spring 1997, Seite 109 bis 115) bekannt und können unter dem Begriff Olefin-Konversionsverfahren zusammengefasst werden. Die Wirtschaftlichkeit solcher Verfahren hängt wesentlich von der erreichten Ausbeute an den gewünschten Olefinen ab.

Der vorliegenden Erfindung liegt die Aufgabe zugrunde, ein Verfahren der eingangs genannten Art sowie eine Vorrichtung zur Durchführung des Verfahrens so auszugestalten, dass eine Erhöhung der Ausbeute an den gewünschten Olefinen mit bis zu drei Kohlenstoffatomen ermöglicht wird.

Diese Aufgabe wird verfahrensseitig erfindungsgemäß dadurch gelöst, dass der Olefinkonversionsstufe eine Paraffin-Olefin-Trennstufe vorgeschaltet ist, in der Olefine und Paraffine voneinander getrennt werden, wobei zumindest ein Teil der Paraffine der Behandlungsanlage zugeführt oder abgezogen und einer Verwertung zugeführt wird, während zumindest ein Teil der Olefine zur Olefinkonversionsstufe geführt wird.

Üblicherweise dient als Behandlungsanlage zur Olefinherstellung eine Olefinanlage mit einem Spaltofen zur Spaltung der Kohlenwasserstoffe und einer nachgeschalteten Fraktionierstufe zur Auftrennung der Spaltprodukte in einzelne Fraktionen. Es können aber auch bestimmte Produktströme einer Raffinerie zur Olefinherstellung genutzt werden, um z. B. zusätzliche Verwertungsmöglichkeiten für die Raffinerie zu erschließen.

Der Grundgedanke der Erfindung besteht darin, das zur Olefin-Konversionsstufe zurückzuführende Kohlenwasserstoffspektrum zuvor in Paraffine und Olefine aufzutrennen und nur die Olefine zur Olefin-Konversionsstufe zu leiten. Dadurch kann die Ausbeute an den gewünschten Olefinen erhöht werden. Auf der anderen Seite bilden die abgetrennten Paraffine einen geeigneten Einsatz für den Spaltofen der Olefinanlage, weshalb diese vorzugsweise dem Spaltofen zugeführt werden. Alternativ oder zusätzlich können die Paraffine auch abgezogen und einer anderen Verwertung zugeführt werden. Insgesamt wird dadurch sowohl die Wirtschaftlichkeit der gesamten Olefinanlage bzw. Raffinerie als auch die Ausbeute an den gewünschten Olefinen erhöht.

Gemäß einer besonders bevorzugten Ausführungsform der Erfindung ist der Olefinkonversionsstufe eine Konversionsproduktfraktionierstufe nachgeschaltet, in der mindestens eine Fraktion erzeugt wird, die Kohlenwasserstoffe mit vier und/oder fünf Kohlenstoffatomen umfasst und zur Olefinkonversionsstufe zurückgeführt wird. Außerdem wird mindestens eine zweite Fraktion erzeugt, die Kohlenwasserstoffe mit mindestens fünf Kohlenstoffatomen umfasst und abgezogen wird. Die Konversionsproduktfraktionierstufe erzeugt somit insgesamt drei Fraktionen, nämlich eine Fraktion, die Kohlenwasserstoffe mit höchstens drei Kohlenstoffatomen umfasst und zur Behandlungsanlage, z. B. zur Fraktionierstufe der Olefinanlage, zurückgeführt wird, eine Fraktion, die Kohlenwasserstoffe mit vier und/oder fünf Kohlenstoffatomen umfasst, und eine Fraktion, die Kohlenwasserstoffe mit mindestens fünf Kohlenstoffatomen umfasst. Die in der Paraffin-Olefin-Trennstufe anfallenden Olefine können entweder direkt der Olefinkonversionsstufe zugeführt werden oder unter Umgehung der Olefinkonversionsstufe zunächst der Konversionsproduktfraktionierstufe aufgegeben werden, so dass sie anschließend über eine Olefinrückführung in die Olefinkonversionsstufe gelangen.

Eine Weiterbildung des Erfindungsgedankens sieht vor, dass in der Paraffin-Olefin-Trennstufe eine Auftrennung in mindestens drei Fraktionen erfolgt, wobei eine erste Fraktion erzeugt wird, die Paraffine mit vier und/oder fünf Kohlenstoffatomen umfasst und der Behandlungsanlage, z. B. dem Spaltofen der Olefinanlage, zugeführt wird, eine zweite Fraktion erzeugt wird, die Olefine mit vier und/oder fünf Kohlenstoffatomen umfasst und zur Olefinkonversionsstufe geführt wird, und eine dritte Fraktion erzeugt wird, die Kohlenwasserstoffe mit mindestens fünf Kohlenstorfatomen umfasst und abgezogen wird. Bei dieser Variante der Erfindung kann die Konversionsproduktfraktionierstufe einfacher ausgestaltet sein, da lediglich eine Fraktion, die Kohlenwasserstoffe mit höchstens drei Kohlenstoffatomen umfasst, abgetrennt werden muss. Die Paraffin-Olefin-Trennstufe wird in diesem Fall so ausgeführt, dass eine Auftrennung in die drei genannten Produktströme ermöglicht wird.

Zweckmäßigerweise umfasst die Paraffin-Olefin-Trennstufe eine Extraktivdestillation. Diese Extraktivdestillation ist vorzugsweise so ausgebildet, dass die genannten drei Produktströme erzeugt werden können.

Die Olefinkonversionsstufe umfasst bevorzugt eine katalytische Umsetzung, z. B. ein katalytisches Spalten (Cracken), der längerkettigen Kohlenwasserstoffe in kürzerkettige Kohlenwasserstoffe. Derartige katalytische Konversionsverfahren sind an sich aus dem Stand der Technik bekannt.

Die Erfindung betrifft femer eine Vorrichtung zur Olefinherstellung aus einem kohlenwasserstoffhaltigen Einsatz mit einer Behandlungsanlage, insbesondere einer Raffinerie oder Olefinanlage, welche eine Fraktioniereinrichtung zur Erzeugung von verschiedenen kohlenwasserstoffhaltigen Fraktionen aufweist, wobei die Fraktioniereinrichtung mit einer Olefinkonversionseinrichtung zur Umsetzung von längerkettigen Olefinen in kürzerkettige Olefine in Verbindung steht.

Vorrichtungsseitig wird die gestellte Aufgabe dadurch gelöst, dass der Olefinkonversionseinrichtung eine Paraffin-Olefin-Trenneinrichtung zur Auftrennung von Olefinen und Paraffinen vorgeschaltet ist, die über eine Paraffinleitung mit der Behandlungsanlage und über eine Olefinleitung mit der Olefinkonversionseinrichtung in Verbindung steht.

Als Behandlungsanlage zur Herstellung der Olefine kann eine Raffinerie oder Olefinanlage dienen. Im Falle einer Olefinanlage ist ein Spaltofen zur Spaltung der Kohlenwasserstoffe vorgesehen, dem eine Fraktioniereinrichtung zur Auftrennung der Spaltprodukte in Fraktionen nachgeschaltet ist. Die Paraffin-Olefin-Trenneinrichtung steht in diesem Fall über die Paraffinleitung mit dem Spaltofen der Olefinanlage in Verbindung.

Die Paraffin-Olefin-Trenneinrichtung ist zweckmäßigerweise mit Destillationskolonnen ausgestattet, die für eine Extraktivdestillation ausgebildet sind. Es sind mindestens zwei Destillationskolonnen vorgesehen, wobei eine die Auftrennung von Paraffinen und Olefinen bewirkt, während die zweite Destillationskolonne als Regeneriersäule zur Rückgewinnung des Extraktionsmittels ausgebildet ist.

Gemäß einer Weiterbildung des Erfindungsgedankens sind mindestens drei Destillationskolonnen vorgesehen, wodurch eine zusätzliche Abtrennung von Kohlenwasserstoffen mit mehr als fünf Kohlenstoffatomen ermöglicht wird. In diesem Fall kann die Konversionsproduktfraktioniereinrichtung einfacher ausgebildet sein, da lediglich die kurzkettigen Kohlenwasserstoffe mit weniger als drei Kohlenstoffatomen abgetrennt werden müssen.

Die Olefinkonversionseinrichtung weist bevorzugt einen katalytischen Reaktor, insbesondere einen Festbettreaktor, auf. Als Katalysatoren können die im Stand der Technik bekannten Katalysatortypen, insbesondere ein Zeolithkatalysator, verwendet werden.

Mit der Erfindung gelingt es insbesondere, die Propylenausbeute in Olefinanlagen oder Raffinerien bei vertretbarem technischen Aufwand deutlich zu steigern. Durch den zusätzlichen Verfahrensschritt der Paraffin-Olefin-Trennung wird die Ausbeute an Zielprodukten in Olefinanlagen wesentlich erhöht. Gleichzeitig werden im Kreislauf geführte Stoffströme minimiert, wodurch die spezifischen Investitionskosten für die Propylenerzeugung reduziert werden. Die Mehrinvestitionen für den zusätzlichen Verfahrensschritt der Paraffin-Olefin-Trennung werden dadurch kompensiert. Insgesamt wird die Wirtschaftlichkeit der Olefinanlage bzw. der Raffinerie erhöht.

Im Folgenden soll die Erfindung anhand eines Vergleichs des Standes der Technik mit in den Figuren schematisch dargestellten Ausführungsbeispielen der Erfindung näher erläutert werden.

Es zeigen
- Figur 1: ein Fließschema einer Olefinanlage mit integrierter Olefin-Konversionsstufe nach dem Stand der Technik
- Figur 2: eine Olefinanlage mit integrierter Olefin-Konversionsstufe und vorgeschalteter Paraffin-Olefin-Trennstufe
- Figur 3: eine Olefinanlage mit integrierter Olefinkonversionsstufe mit Konversionsproduktfraktionierstufe und vorgeschalteter Paraffin-Olefin-Trennstufe mit direkter Olefinzufuhr zur Olefinkonversionsstufe
- Figur 4: wie Figur 3, jedoch mit indirekter Olefinzufuhr zur Olefinkonversionsstufe
- Figur 5: ein Fließschema einer Olefinanlage mit integrierter Olefin-Konversionsstufe mit Konversionsproduktfraktioniereinrichtung und vorgeschalteter Paraffin-Olefin-Trennstufe mit integrierter Fraktionierung
- Figuren 6 und 7: verschiedene Ausgestaltungen der Paraffin-Olefin-Trennstufe.

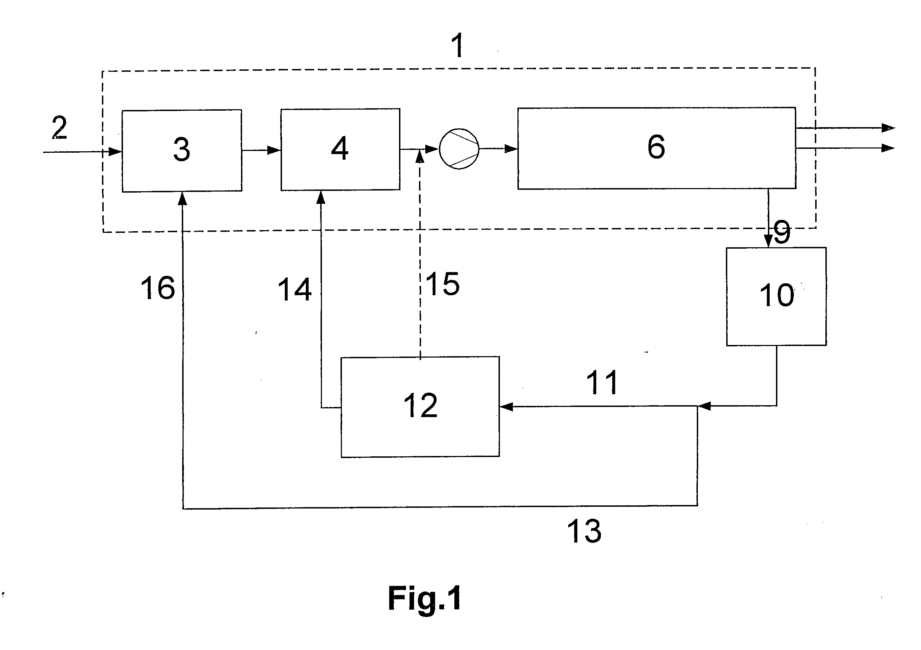

Die in Figur 1 dargestellte Olefinanlage 1 nach dem Stand der Technik wird mit Naphtha betrieben, welches über Leitung 2 einem Spaltofen 3 zugeführt wird. Es können auch mehrere Spaltöfen vorgesehen sein, welche aus dem Naphtha ein Spaltgas erzeugen, das einer Wasserwäsche 4 zugeführt und mittels eines Verdichters 5 einer Fraktionsstufe 6 zugeführt wird. In der Fraktionierstufe 6 werden Olefine, Pyrolysebenzin, Pyrolyseöl und leichte Gase über Leitungen 7 und 8 abgezogen. In der Fraktionierstufe 6 anfallende Kohlenwasserstoffe mit vier oder fünf Kohlenstoffatomen werden über Leitung 9 einer Dien/Acetylen-Entfernung 10 zugeführt. Die verbleibenden Paraffine und Olefine mit vier und/oder fünf Kohlenstoffatomen werden über Leitung 11 in eine Olefin-Konversionsstufe 12 eingeleitet. Die Olefin-Konversionsstufe 12 ist üblicherweise als katalytischer Festbettreaktor ausgebildet und setzt die längerkettigen Olefine (C₄/C₅-Olefine) in kürzerkettige Olefine (C₂ - C₄- Olefine) um. Die Butane werden über Leitung 13 ausgeschleust und direkt dem Spaltofen 3 als Einsatzstoff zugeführt, wo sie zu Olefinen und Nebenprodukten gespalten werden. Die in der Olefin-Konversionsstufe 12 erzeugten Olefine mit zwei bis vier Kohlenstoffatomen sowie anfallendes Benzin und leichte Gase werden über Leitung 14 der Wasserwäsche 4 der Olefinanlage 1 zugeführt. Ein Teilstrom kann auch direkt über Leitung 15 vor die Pumpe 5 geführt werden. Diese Anordnung gibt die einfachste Variante nach dem Stand der Technik zur Steigerung der Propylenausbeute in Olefinanlagen wieder.

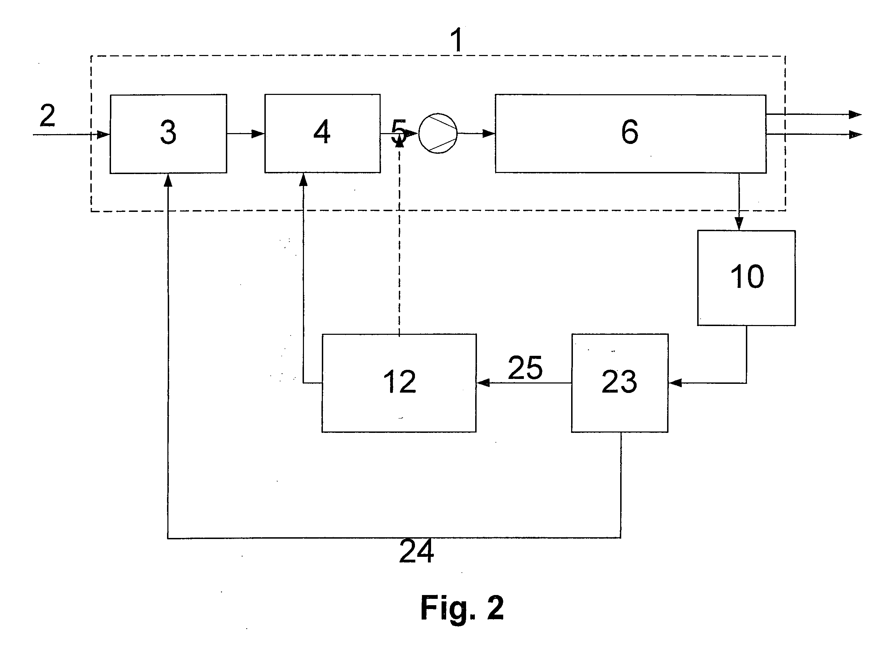

Figur 2 zeigt eine Ausgestaltung der Erfindung, die auf der in Figur 1 dargestellten Anlage nach dem Stand der Technik aufbaut. Dieselben Anlagenteile sind mit denselben Bezugsziffern bezeichnet. Diese Variante der Erfindung unterscheidet sich von dem Stand der Technik dadurch, dass der Olefinkonversionsstufe 12 eine Paraffin-Olefin-Trennstufe 23 vorgeschaltet ist. Dieser zusätzliche Verfahrensschritt dient dazu, die Paraffine von den Olefinen zu trennen. Während die Paraffine (Butan, Pentan) über Leitung 24 dem Spaltofen 3 der Olefinanlage 1 als Einsatzstoff zugeführt werden, werden die Olefine (Buten, Penten) über eine Olefinleitung 25 zur Olefin-Konversionsstufe 12 geführt. Dadurch kann eine wesentliche Steigerung der Propylen-Ausbeute der Olefinanlage erzielt werden.

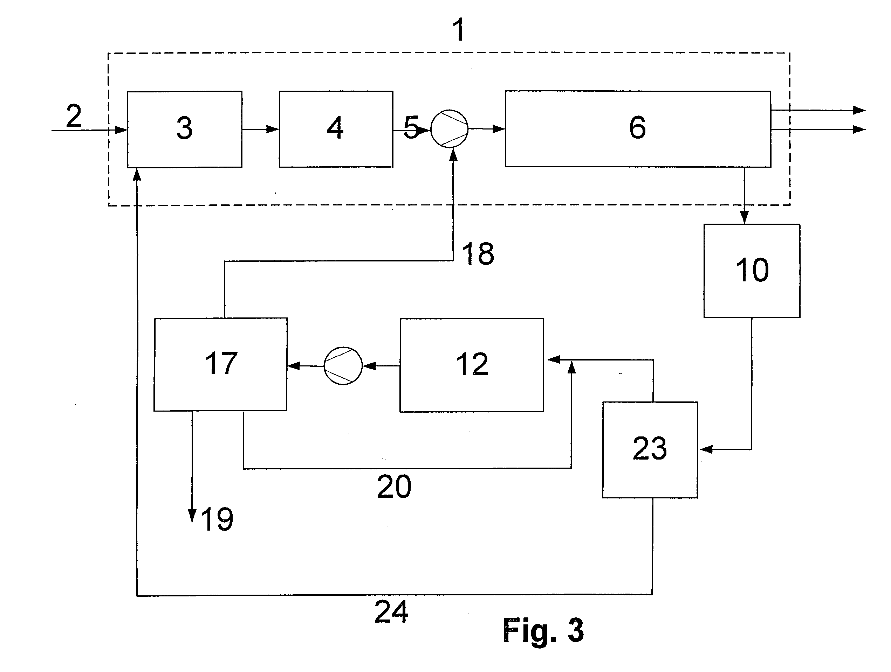

Die in Figur 3 gezeigte Ausführung unterscheidet sich von der in Figur 2 gezeigten dadurch, dass der Olefinkonversionsstufe 12 eine Konversionsproduktfraktionierstufe nachgeschaltet ist, in der die in der Olefinkonversionsstufe 12 erzeugten Kohlenwasserstoffe in verschiedene Fraktionen aufgetrennt werden. Die Kohlenwasserstoffe mit höchstens drei Kohlenstoffatomen werden über Leitung 18 direkt zur Fraktionierstufe 6 der Oberfläche 1 zurückgeführt, während die längerkettigen Kohlenwasserstoffe mit mindestens fünf Kohlenstoffatomen und Benzinprodukt über Leitung 19 abgeführt, und die Kohlenwasserstoffe mit vier und/oder fünf Kohlenstoffatomen über Leitung 20 von der Konversionsproduktfraktionierstufe 17 abgezogen und zur Olefinkonversionsstufe 12 zurückgeführt werden.

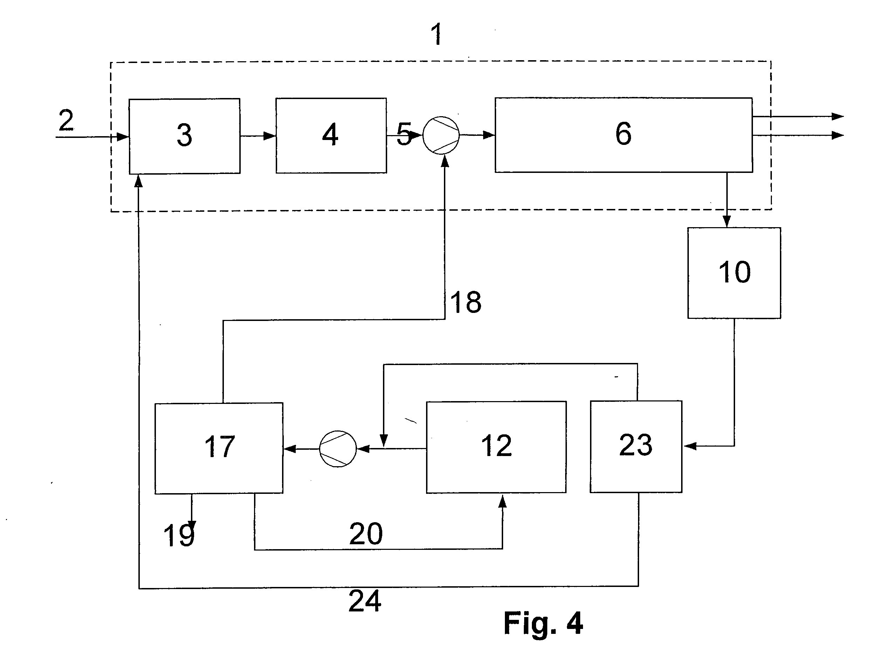

Figur 4 zeigt eine Alternative zu der in Figur 3 gezeigten Ausführungsform, wobei die in der Paraffin-Olefin-Trennstufe 23 anfallenden Olefine nicht direkt der Olefinkonversionsstufe 12 zugeführt werden, sondem indirekt über die Konversionsproduktfraktioniereinrichtung und Olefin-Rückführung 20.

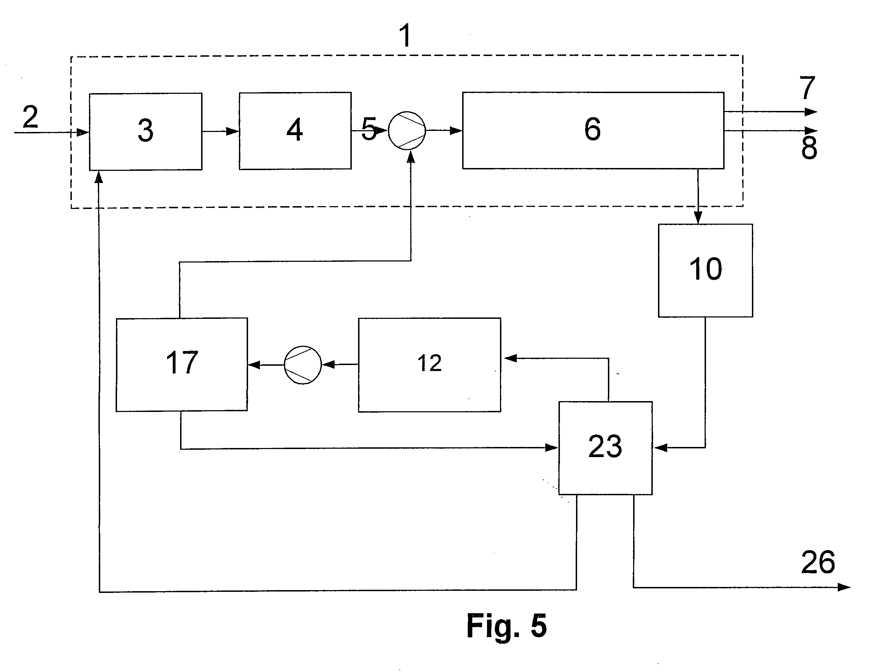

In Figur 5 ist eine andere Variante der Erfindung dargestellt, bei der die Paraffin-Olefin-Trennstufe 23 gleichzeitig als Fraktionierstufe ausgebildet ist. Dadurch kann die in Figur 3 gezeigte Abtrennung von Kohlenwasserstoffen mit mehr als fünf Kohlenstoffatomen in der Konversionsproduktfraktionierstufe 17 entfallen. Diese längerkettigen Kohlenwasserstoffe werden somit nicht, wie in Figur 3 gezeigt, über Leitung 19, sondern von der Propylen-Olefin-Trennstufe 23 über Leitung 26 abgeführt. Die übrigen Verfahrensschritte entsprechen der in der Figur 3 gezeigten Variante.

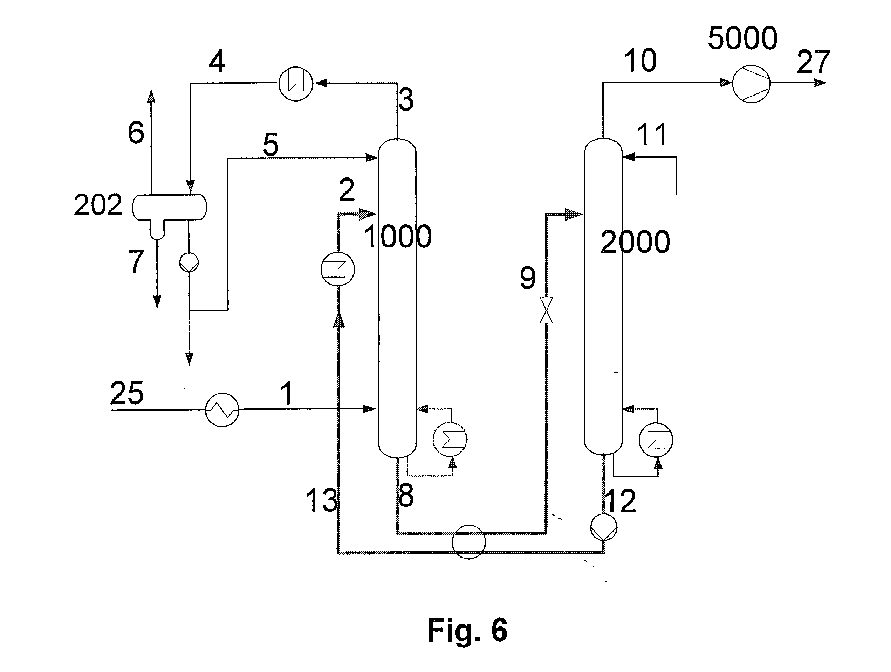

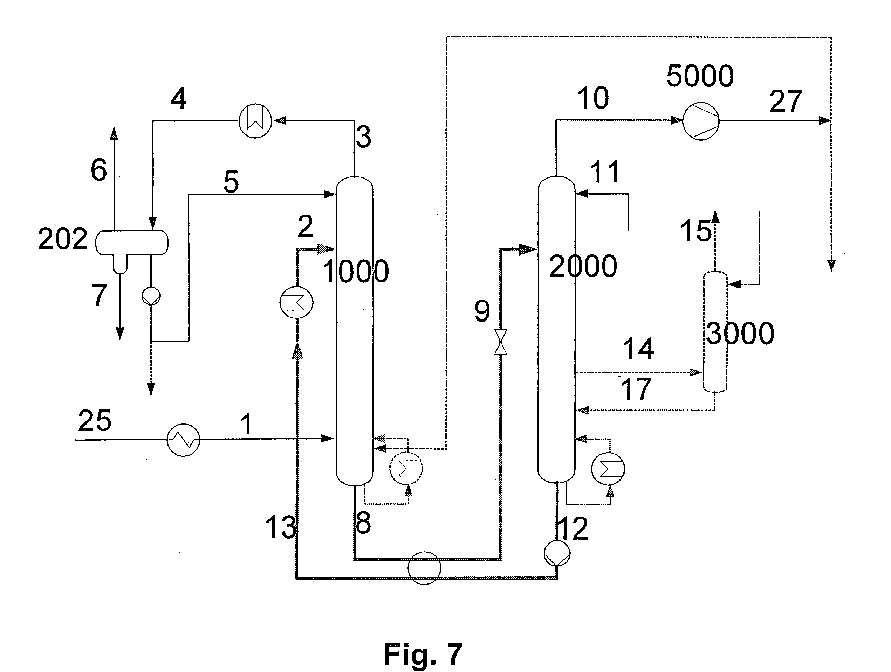

Die Figuren 6 und 7 betreffen Detailansichten der Paraffin-Olefin-Trennstufe. Die Paraffin-Olefin-Trennstufe ist jeweils als Extraktivdestillation ausgebildet.

Die in Figur 6 gezeigte Paraffin-Olefin-Trennstufe wird über Leitungen 25 und 1 mit einem Gemisch aus Butan und Pentan sowie Buten und Penten, welches von der Konversionsproduktfraktioniereinrichtung abgezogen wird, beschickt. Dieses Gemisch wird in eine erste Destillationskolonne 1000 eingeleitet. Die Destillationskolonne 1000 wird mit einem Extraktionsmittel betrieben, welches über Leitungen 12, 13 und 2 in den oberen Teil der Destillationskolonne 1000 eingeleitet wird. Das Extraktionsmittel kann aus den folgenden Substanzen bestehen: NMP (N-Methyl-Pyrolidon), DMF (Dimethylformamid), NFM (N-Formylmorpholine), Aceton, Acetonnitril, Furfural, DMAC (Dimethylacetamid), Sulpholan, Diethylenglykol (Glykolmischungen), oder Dimethylsulfoxid. Es können auch Lösemittelgemische und als Additive Wasser oder Methanol verwendet werden. Vom Kopf der Destillationskolonne 1000 werden die Paraffine Butan und/oder Pentan über Leitung 3 abgezogen und schließlich über Leitung 6 zum Spaltofen der Olefinanlage zurückgeführt. Ein Teil dieser Paraffine wird über Leitung 5 zur Destillationskolonne 1000 zur Rückwaschung zurückgeführt. Über Leitung 7 kann Wasser abgetrennt werden. Die im Sumpf der Destillationskolonne 1000 anfallenden Olefine Buten und Penten werden über Leitung 8 und einen Wärmetauscher 104 und Leitung 9 einer zweiten Destillationskolonne 2000 zugeführt, die als Regeneriersäule dient. Zur Rückwaschung wird über Leitung 11 Wasser oder ein extemer Rücklauf dem Kopf der Destillationskolonne 2000 zugeführt. Die Olefine Buten und Penten werden schließlich über Leitung 10 vom Kopf der Destillationskolonne 2000 abgezogen und über einen Kompressor 5000 und Leitung 27 zur Olefinkonversionsstufe zurückgeleitet. In der Destillationskolonne 1000 wird im vorliegenden Ausführungsbeispiel ein Druck von 9 bar eingestellt, der für eine Weiterverarbeitung der Paraffine im Spaltofen ausreichend ist. Sofern die Paraffine lediglich zur Verflüssigung oder zur Einspeisung in ein Heizgasnetz vorgesehen sind, reicht ein Druck in der Destillationskolonne 1000 von 4 bis 6 bar aus. In der Destillationskolonne 2000 wird ein möglichst niedriger Druck eingestellt, um eine Zersetzung des Extraktionsmittels zu vermeiden. Daher beträgt der, Druck im vorliegenden Ausführungsbeispiel 1 bis 4 bar, insbesondere weniger als 2 bar. Sofern ein Hilfsstoff zugeführt wird (z.B. höhere Kohlenwasserstoffe), sollte der Druck so hoch sein, dass eine Kondensation oder gasförmige Kopfproduktabgabe gewährleistet werden kann. In diesem Fall ist ein Druck von 2 bis 6 bar vorgesehen (Kondensation: 4 bis 5 bar, gasförmiges Kopfprodukt: > 3 bar). Die Temperaturen in den Destillationskolonnen 1000 und 2000 werden den jeweiligen Drücken und der gestellten Trennaufgabe angepasst. In der Regeneriersäule 2000 wird eine Siedetemperatur im Sumpf eingestellt, die ausreichend unterhalb der Zersetzungstemperaturen der Lösemittel ist.

In Figur 7 sind dieselben Anlagenteile wie in Figur 6 mit denselben Bezugsziffern bezeichnet. Die in Figur 7 dargestellte Variante unterscheidet sich von der in Figur 6 gezeigten durch eine zusätzliche Maßnahme zur Acethylenausschleusung mit einer dritten Destillationskolonne 3000, die über Leitungen 14 und 17 mit der Destillationskolonne 2000 verbunden ist. Über Leitung 15 wird vom Kopf der Destillationskolonne 2000 Acethylen abgezogen. Über Leitung 16 findet eine Rückwaschung mit Wasser oder einem externen Rücklauf statt.

## Patentansprüche

1. Verfahren zur Olefinherstellung aus einem kohlenwasserstoffhaltigen Einsatz, wobei der Einsatz einer Behandlungsanlage (1), insbesondere einer Raffinerie oder Olefinanlage zugeführt wird, in der verschiedene kohlenwasserstoffhaltige Fraktionen erzeugt werden, wobei zumindest ein Teil der Fraktionen, die längerkettige, insbesondere mindestens vier Kohlenstoffatome aufweisende, Olefine enthalten, einer Olefinkonversionsstufe (12) zugeführt wird, in der zumindest ein Teil der längerkettigen Olefine in kürzerkettige Olefine umgesetzt wird, welche zumindest zum Teil zur Behandlungsanlage (1) zurückgeführt werden, **dadurch gekennzeichnet, dass** der Olefinkonversionsstufe (12) eine Paraffin-Olefin-Trennstufe (23) vorgeschaltet ist, in der Olefine und Paraffine voneinander getrennt werden, wobei zumindest ein Teil der Paraffine der Behandlungsanlage (1) zugeführt oder abgezogen und einer Verwertung zugeführt wird, während zumindest ein Teil der Olefine zur Olefinkonversionsstufe (12) geführt wird.

2. Verfahren nach Anspruch 1, wobei die Behandlungsanlage (1) als Olefinanlage mit einem Spaltofen (3) zur Spaltung der Kohlenwasserstoffe und einer Franktionierstufe (6) zur Auftrennung der Spaltprodukte in Franktionen betrieben wird, **dadurch gekennzeichnet, dass** zumindest ein Teil der in der Olefin-Trennstufe (23) von den Olefinen abgetrennten Paraffine dem Spaltofen (3) der Olefinanlage zugeführt wird.

3. Verfahren nach Anspruch 1 oder 2, **dadurch gekennzeichnet, dass** der Olefinkonversionsstufe (12) eine Konversionsproduktfraktionierstufe (17) nachgeschaltet ist, in der mindestens eine Fraktion erzeugt wird, die Kohlenwasserstoffe mit vier und/oder fünf Kohlenstoffatomen umfasst und zur Olefinkonversionsstufe (12) zurückgeführt wird und mindestens eine zweite Fraktion erzeugt wird, die Kohlenwasserstoffe mit mindestens fünf Kohlenstoffatomen umfasst und abgezogen wird.

4. Verfahren nach Anspruch 3, **dadurch gekennzeichnet, dass** die in der Paraffin-Olefin-Trennstufe (23) anfallenden Olefine unter Umgehung der Olefinkonversionsstufe (12) direkt der Konversionsproduktfraktionierstufe (17) zugeführt werden.

5. Verfahren nach Anspruch 1 oder 2, **dadurch gekennzeichnet, dass** in der Paraffin-Olefin-Trennstufe (23) eine Auftrennung in mindestens drei Fraktionen erfolgt, wobei eine erste Fraktion erzeugt wird, die Paraffine mit vier und/oder fünf Kohlenstoffatomen umfasst und der Behandlungsanlage (1) zugeführt wird, eine zweite Fraktion erzeugt wird, die Olefine mit vier und/oder fünf Kohlenstoffatomen umfasst und zur Olefinkonversionsstufe (12) geführt wird, und eine dritte Fraktion erzeugt wird, die Kohlenwasserstoffe mit mindestens fünf Kohlenstoffatomen umfasst und abgezogen wird.

6. Verfahren nach einem der Ansprüche 1 bis 5, **dadurch gekennzeichnet, dass** die Paraffin-Olefin-Trennstufe (23) eine Extraktivdestillation umfasst.

7. Verfahren nach einem der Ansprüche 1 bis 6, **dadurch gekennzeichnet, dass** die Olefinkonversionsstufe (12) eine katalytische Umsetzung der längerkettigen Kohlenwasserstoffe in kürzerkettige Kohlenwasserstoffe umfasst.

8. Vorrichtung zur Olefinherstellung aus einem kohlenwasserstoffhaltigen Einsatz mit einer Behandlungsanlage (1), insbesondere einer Raffinerie oder Olefinanlage, welche eine Fraktioniereinrichtung (6) zur Erzeugung von verschiedenen kohlenwasserstoffhaltigen Fraktionen aufweist, wobei die Fraktioniereinrichtung (6) mit einer Olefinkonversionseinrichtung (12) zur Umsetzung von längerkettigen Olefinen in kürzerkettige Olefine in Verbindung steht, **dadurch gekennzeichnet, dass** der Olefinkonversionseinrichtung (12) eine Paraffin-Olefin-Trenneinrichtung (23) zur Auftrennung von Olefinen und Paraffinen vorgeschaltet ist, die über eine Paraffinleitung mit der Behandlungsanlage (1) und über eine Olefinleitung mit der Olefinkonversionseinrichtung (12) in Verbindung steht.

9. Vorrichtung nach Anspruch 8, wobei die Behandlungsanlage (1) als Olefinanlage mit einem Spaltofen (3) zur Spaltung der Kohlenwasserstoffe und einer Fraktioniereinrichtung (6) zur Auftrennung der Spaltprodukte in Fraktionen ausgebildet ist, **dadurch gekennzeichnet, dass** die Paraffin-Olefin-Trenneinrichtung (23) über die Paraffinleitung mit dem Spaltofen (3) der Olefinanlage in Verbindung steht.

10. Vorrichtung nach Anspruch 8 oder 9, **dadurch gekennzeichnet, dass** die Paraffin-Olefin-Trenneinrichtung (23) für eine Extraktivdestillation ausgebildete Destillationskolonnen aufweist.

11. Vorrichtung nach einem der Ansprüche 8 bis 10, **dadurch gekennzeichnet, dass** die Olefinkonversionseinrichtung (12) einen katalytischen Reaktor, insbesondere einen Festbettreaktor, aufweist.
